Europäisches Patentamt

(19) European Patent Office (11) Numéro de publication: **0 074 290**

Office européen des brevets **B1**

(12) **FASCICULE DE BREVET EUROPÉEN**

(45) Date de publication du fascicule du brevet: 23.07.86 (51) Int. Cl.⁴: **C 12 M 1/00**

(21) Numéro de dépôt: **82401418.7**

(22) Date de dépôt: **29.07.82**

(54) **Procédé et installation pour la réalisation d'une dégradation en milieu anaérobie de produits, sous-produits et des déchets organiques.**

(30) Priorité: **29.07.81 FR 8114740**
**22.07.82 FR 8212829**

(43) Date de publication de la demande:
**16.03.83 Bulletin 83/11**

(45) Mention de la délivrance du brevet:
**23.07.86 Bulletin 86/30**

(84) Etats contractants désignés:
**BE CH DE GB IT LI NL SE**

(56) Documents cité:
**FR-A-818 680**
**FR-A-1 048 146**
**FR-A-1 146 820**
**FR-A-2 305 113**
**FR-A-2 353 638**
**GB-A-695 322**
**US-A-4 053 395**

(73) Titulaire: **UNION INDUSTRIELLE ET D'ENTREPRISE, 32 avenue Hoche, F-75008 Paris (FR)**
Titulaire: **Ducellier, Gilbert, Colline de L'Estanove route de Laveurune, Bat. D 2, F-34000 Montpellier (FR)**
Titulaire: **Pavia, André, 408 rue Valéry Larbaud, F-34100 Montpellier (FR)**

(72) Inventeur: **Ducellier, Gilbert, Colline de l'Estanove Route de Laveurune, Bat. Dé, 34000 Montpellier (FR)**
Inventeur: **Pavia, André, 408 rue Valéry Larbaud, 34100 Montpellier (FR)**

(74) Mandataire: **Beauchamps, Georges, Cabinet Z.Weinstein 20, avenue de Friedland, F-75008 Paris (FR)**

## Description

La présente invention a pour objet un procédé et une installation pour réaliser la dégradation en milieu anaérobie de produits, sous-produits et déchets organiques d'origine humaine, animale et/ou végétale.

L'invention a plus particulièrement pour objet un procédé et une installation pour réaliser une méthanisation en continu de composés organiques solides et/ou liquides.

Les matières organiques de diverse origine telle que, humaine, animale ou végétale, peuvent conduire par des dégradations microbiologiques successives ou simultanées en milieu anaérobie, à des composés gazeux dont les plus importants sont le méthane et le dioxyde de carbone.

La nature et la composition de ces produits organiques sont très variables et ils sont composés d'éléments plus ou moins polymérisés et imbriqués. Ces produits organiques constituent la plupart du temps un substrat solide, fibreux ou non, à forte teneur en matières sèches. Ceci est notamment le cas des déchets urbains, agricoles, par exemple.

Mais, ces produits peuvent être également liquides avec de faibles teneurs en matières sèches telles que par exemple les boues de station d'épuration, les lisiers, le sérum de lait.

La grande majorité de ces substrats est constituée par des composés ternaires tels que sucres, amidons, hémicelluloses, celluloses et lignines, mais des corps quaternaires comme les protides et les peptides peuvent aussi y être associés.

Les agents participants à ces dégradations proviennent généralement d'habitats d'origine à la fois animale, végétale et tellurique. De nombreuses espèces ont été dénombrées. Elles évoluent dans des milieux à pH et à potentiel d'oxydo - réduction très variables avec des plages différentes suivant les espèces psychrophiles, mésophiles ou thermophiles.

De manière simplifiée, la dégradation en milieu anaérobie d'une matière organique, et notamment d'une matière organique insoluble fait intervenir trois phases:

- une phase d'hydrolyse au cours de laquelle les enzymes transforment les composés insolubles en corps solubles;

- une phase acidogène au cours de laquelle les matières organiques dissoutes sont transformées en composés tels que acide gras, alcool ou autres. Dans cette phase acidogène on peut distinguer une phase acétogène au cours de laquelle est produit de l'acide acétique qui est un des composés essentiels pour la formation du méthane au cours de la dernière phase ci-dessous;

- une phase méthanogène au cours de laquelle le méthane et le dioxyde de carbone sont produits.

On connaît déjà des techniques de fermentation consistant à utiliser des cuves de digestion fonctionnant en continu ou en discontinu. Les cuves fonctionnant en discontinu sont surtout utilisées pour les produits organiques ou substrats solides et hétérogènes et ne sont pas pratiques ni rentables industriellement. Les cuves de digestion fonctionnant en continu sont presque essentiellement utilisées avec des effluents liquides faiblement chargés en matières sèches. En effet, dans ces installations, le traitement de substrat solide, hétérogène ou non, présente de nombreuses difficultés liées notamment à la circulation et l'homogénéisation du substrat dans la cuve ce qui induit des mauvaises conditions de fonctionnement et perturbe fortement les processus de dégradation de la matière organique. Les rendements de tellas installations sont relativement faibles. Pour assurer une méthanisation dans des conditions convenables, il était donc nécessaire d'utiliser un substrat liquide ou fortement dilué par addition d'eau. Cette quantité d'eau associée au substrat à traiter entraîne des rejets liquides polluants et l'utilisation de cuves de volume important.

Par le document FR-A-2 305 113, on connaît par exemple une installation de digestion de matières organiques dans laquelle la matière à travailler est introduite, après avoir été humidifiée, dans un compartiment cylindrique dans lequel elle subit une fermentation aérobie souspression, puis elle est poussée par un piston tasseur, à travers un siphon coudé, dans un petit compartiment de fermentation anaérobie puis dans un grand compartiment. Dans le coude de siphon, des fourches s'opposent à un reflux de la matière. Le brevet précité prévoit également un puits d'extraction dans lequel la matière fermentée est entraînée par une griffe.

On connaît aussi par le document FR-A-1 048 146 une installation de fermentation anaérobie selon laquelle la matière fermentescible est maintenue prisonnière entre des claies B et C.

Une telle installation ne se prête pas à une fermentation au niveau industriel qui exige une circulation de la matière fermentescible.

Enfin, on connaît aussi par le document EP-A-33 724 une installation de fermentation anaérobie dans laquelle la matière fermentescible circule à l'aide de l'emploi d'un dispositif déplaçable compliqué 4, 5 formant une chambre 6 d'accumulation de biogaz. Cette installation n'apparaît pas appropriée pour la circulation de matière à teneur élevée en matières sèches.

La présente invention a pour but de remédier à ces difficultés en proposant un procédé et une installation pour assurer la fermentation anaérobie de produits, sous-produits ou déchets organiques d'origine humaine, animale, végétale notamment fortement chargés en matières sèches, hétérogènes ou non, en assurant une bonne homogénéisation des produits dans la cuve de digestion pour ainsi favoriser d'une part le processus de dégradation de la matière organique et d'autre part la circulation des produits dans la cuve par un effet de fluidisation de la masse des produits.

A cet effet, l'invention propose un procédé pour réaliser une dégradation en milieu anaérobie, par exemple une méthanogénèse, de produits, sous-produits ou déchets organiques d'origine humaine, animale et/ou végétale avantageusement à teneur élevée en produits solides, consistant à alimenter lesdits produits a dégrader dans une enceinte fermée, après avoir éventuellement ensemencé lesdits produits avec un substrat convenable, à imposer auxdits produits un sens de circulation dans ladite enceinte, à recueillir le gaz produit appelé biogaz dégagé au-dessus de ladite masse de produits et à évacuer les produits dégrades, caractérisé en ce qu'on réalise une réintroduction du biogaz dans ladite enceinte, de manière à aboutir à une agitation et/ou homogénéisation et/ou circulation forcée desdits produits.

Ainsi, il est possible de traiter des produits ou substrats fortement chargés en matières sèches, hétérogènes ou non.

Selon une autre caractéristique de l'invention, l'alimentation des produits se fait pneumatiquement en exerçant une poussée pneumatique sur lesdits produits pour les forcer à pénètrer dans l'enceinte, ce qui simplifie l'appareillage et élimine les problèmes de blocage, de destruction, de corrosion liés à des dispositifs mécaniques pour l'alimentation ou l'extraction du substrat.

Selon encore une autre caractéristique de l'invention, on réalise une réintroduction du biogaz dans la masse de produits présents dans l'enceinte, de préférence par émission de jets brefs successifs sous pression.

Selon encore une autre caractéristique de l'invention, on provoque des variations brutales de la pression de gaz contenue dans l'enceinte fermée.

L'invention a également pour objet une installation pour réaliser une dégradation en milieu anaérobie, par exemple une méthanogénèse, de produits, sous produits ou déchets organiques d'origine humaine, animale et/ou végétale, du type comprenant un réacteur ou digesteur ayant une cuve de fermentation anaérobie divisée verticalement en deux parties, une première partie est reliée à un puits d'alimentation, la seconde partie est reliée à un puits d'évacuation des produits fermentés, une sortie de gaz ou biogaz reliée à un récipient formant gazomètre à travers une valve appropriée, et des moyens reliés audit puits d'évacuation pour recueillir les produits fermentés, caractérisée en ce qu'elle comprend des moyens d'introduction de biogaz sous pression dans ladite enceinte de manière à réaliser une agitation et/ou homogénéisation et/ou circulation forcée desdits produits.

Selon une autre caractéristique, la cuve de fermentation est divisée verticalement par une cloison centrale ayant une hauteur inférieure à celle de la cuve. En outre, la cloison précitée séparant la cuve de fermentation en des première et seconde partie présente un passage formant communication entre lesdites première et seconde parties au moins au niveau du fond de la cuve.

Selon encore une autre caractéristique, l'installation comprend un compresseur dont l'entrée est au moins reliée au gazomètre précité dont la sortie est reliée à une pluralité de conduits débouchant dans le fond de la cuve de fermentation ou à un conduit débouchant à un sommet du puits d'alimentation ou à un conduit débouchant dans le fond du puits d'alimentation.

Ces conduits peuvent être équipés avantageusement avec un clapet anti-retour. Eventuellement, les conduits débouchant dans le fond de la cuve de fermentation peuvent être munis d'une soupape pour insuffler le biogaz par jets brefs et successifs.

Par ailleurs, la sortie précitée du compresseur peut être reliée également à un conduit débouchant dans le fond du puits d'évacuation et/ou à un conduit débouchant au sommet du puits d'évacuation, chaque conduit étant équipé avec au moins une vanne.

Ainsi, il est possible de recycler le biogaz dans toutes parties de l'installation.

En outre, l'installation comprend éventuellement un compresseur d'air dont la sortie est reliée au puits d'alimentation précité et/ou au puits d'évacuation précité pour ainsi provoquer ou continuer, si on le désire, une dégradation en milieu aérobie dans le puits d'alimentation ou accélérer le processus de transformation du substrat dans le puits d'évacuation en vue de son utilisation en compost.

L'installation comprend également des moyens pour recycler dans ledit réacteur et/ou dans le puits d'alimentation ou le puits d'évacuation, le liquide séparé des produits solides fermentés recueillis à la sortie du puits précité d'évacuation, ce liquide étant un levain ou un inoculum.

Selon une nouvelle caractéristique de l'invention, le puits d'alimentation possède deux séries de fentes situées à une certaine distance l'une de l'autre et à une certaine distance respectivement du couvercle et de l'extrémité inférieure du puits.

En outre, La valve notamment hydraulique, est située soit en dehors de la cuve soit dans le puits, tandis que le gazomètre se trouve soit en dehors de la cuve soit au-dessus de celle-ci. Dans ce dernier cas, les gaz s'échappent par un tuyau qui traverse la cuve et aboutit à un siphon de condensation destiné à récupérer l'eau présente dans le biogaz provenant de la fermentation. De préférence, ces gaz sont amenés ensuite à un épurateur.

En outre, selon un premier mode de réalisation du réacteur, les puits d'alimentation et d'évacuation précités sontsitués à proximité l'un de l'autre, la cloison précitée étant disposée verticalement entre les deux orifices débouchant desdits puit dans le réacteur, cette cloison ayant une largeur inférieure à la largeur de ladite cuve et une hauteur inférieure à la hauteur de la cuve tandis que le fond de celle-ci présente une double pente qui a avantageusement une forme d'ellipse.

Selon un autre mode de réalisation du réacteur de l'installation, les puits d'alimentation et d'évacuation précités sont disposés de manière sensiblement diamètralement opposée sur le pourtour de la cuve de fermentation précitée, la cloison précitée étant disposée verticalement et selon sensiblement un diamètre de la cuve et ayant une hauteur inférieure à celle de la cuve. Le fond de cette cuve à de préférence une pente unique tandis que la cloison précitée présente un passage à sa partie inférieure.

Enfin, l'installation comprend des échangeurs de chaleur permettant de réaliser différents échanges thermiques entre les fluides circulant dans l'installation et notamment pour réchauffer le levain ou inoculum avant son alimentation dans le réacteur ou digesteur.

L'invention sera mieux comprise et d'autres caractéristiques, détails et avantages de celle-ci apparaîtront plus clairement au cours de la description explicative qui va suivre faite en référence aux dessins schématiques annexés illustrant différents modes de réalisation de l'invention et donnés uniquement à titre d'exemple, et dans lesquels:

La figure 1 est une coupe schématique d'un mode de réalisation du réacteur de l'invention.

La figure 2 est une coupe selon la ligne II-II de la figure 1.

La figure 3 est une coupe schématique illustrant un autre mode de réalisation du réacteur de l'invention. La figure 4 est une coupe selon la ligne IV-IV de la figure 3.

La figure 5 est une vue en coupe schématique avec arrachements partiaux d'un autre mode de réalisation du réacteur de l'invention.

La figure 6 est une vue en coupe selon la ligne VI-VI de la figure 5.

Les figures 7 et 8 sont des plans synoptiques d'un premier mode de réalisation de l'installation.

La figure 9 est un plan synoptique d'un autre mode de réalisation de l'installation.

Les figures 10a, 10b, 11a, 11b représentent schématiquement le réacteur de l'invention pour illustrer les effets du procédé de l'invention sur l'état du substrat contenu dans ledit réacteur.

La figure 12 est une vue schématique illustrant une installation de traitement du substrat fermenté en sortie du réacteur de l'invention.

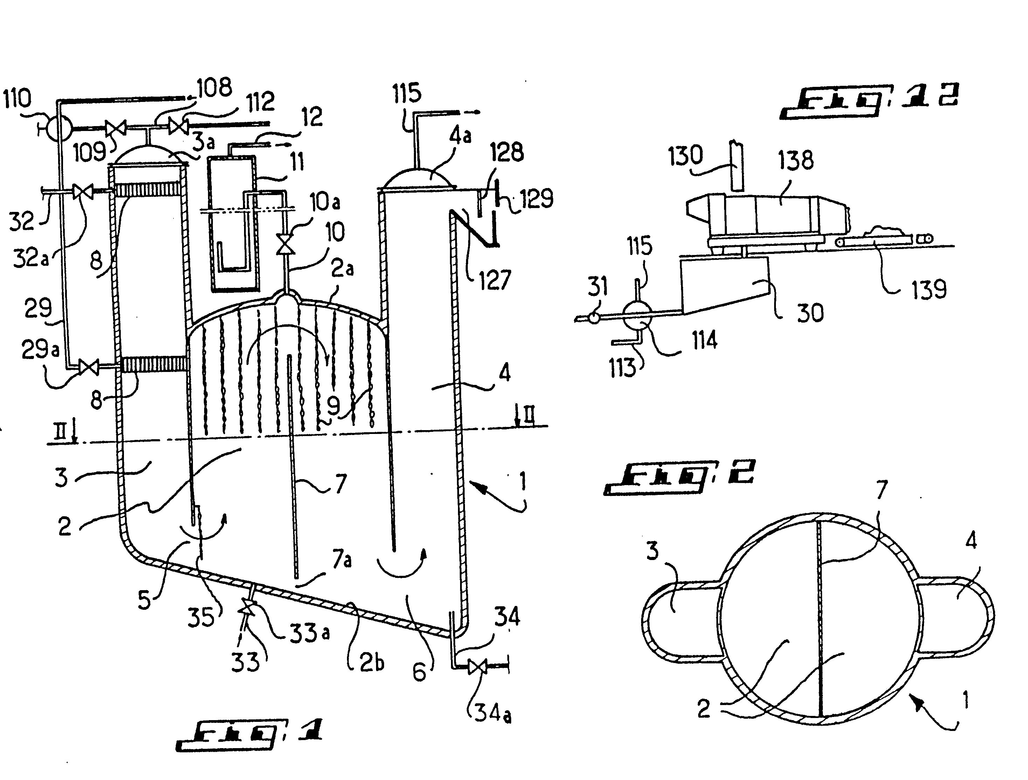

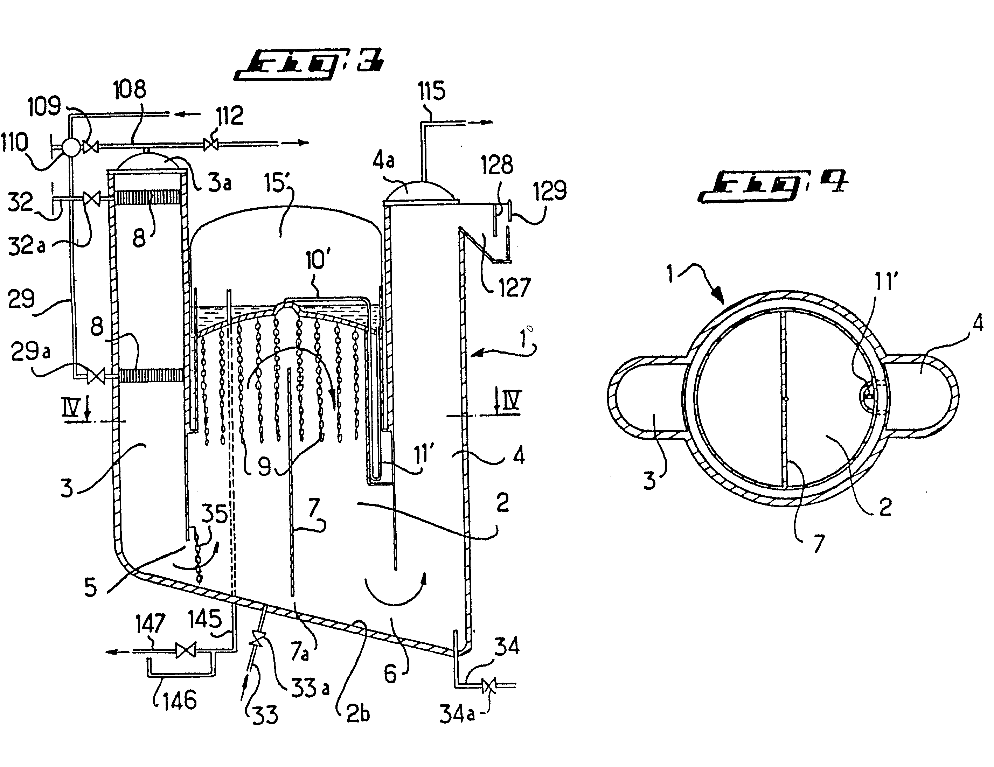

En se référant aux figures 1 et 2, on étudiera un premier mode de réalisation de l'installation et notamment un premier mode de réalisation du réacteur de l'installation.

Le réacteur 1 de l'installation comprend un puits d'alimentation 3 fermé par un couvercle 3a parfaitement étanche, une cuve de fermentation 2 comprenant un couvercle en forme de dôme 2a et un puits d'évacuation 4 fermé par un couvercle 4a. La cuve de fermentation 2 est séparée en deux parties par une cloison 7 située sensiblement selon un diamètre de la cuve 2 et ayant une hauteur inférieure à celle de la cuve 2. De plus, cette cloison 7 laisse un passage 7a à sa partie inférieure.

Dans le mode de réalisation illustré à la figure 1, et comme cela est clairement visible à la figure 2, les puits d'alimentation et d'évacuation 3 et 4 sont situés de manière diamétralement opposée sur le pourtour de la cuve 2 et sont reliés respectivement aux première et seconde parties de la cuve 2 à travers des siphons 5,6. Le siphon 5 peut être éventuellement équipé d'un moyen tel que des chaînes 35 à pliage unidirectionnel freinant tout retour du substrat en cours de fermentation anaérobie vers le puits d'alimentation 3.

La cuve 2 comprend un conduit de sortie de gaz 10 muni d'une vanne 10a et débouchant dans une valve hydraulique 11, le gaz ou biogaz se dégageant ensuite par le conduit 12 vers un gazomètre 15 non représenté à la figure 1.

Le puits d'alimentation comprend également deux séries de fentes 8 espacées l'une par rapport à l'autre sur le puits d'alimentation 3.

Le réacteur 1 comprend également des chaînes 9 suspendues au dôme 2a de la cuve 2 dont le rôle sera décrit ultérieurement.

Dans le mode de réalisation illustré à la figure 1, le puits d'évacuation 4 possède à son sommet un couvercle 4a muni d'un conduit de gaz 115, un siphon de sortie 127 et un déversoir 128 à ouverture réglable 129.

Par ailleurs, le réacteur 1 comprend plusieurs canalisations permettant soit l'évacuation des gaz depuis les puits d'évacuation ou d'alimentation, soit l'introduction d'air dans ces puits d'évacuation ou d'alimentation, soit l'introduction de levain ou inoculum dans la cuve 2. Ces différentes canalisations seront décrites plus en détail lors de la description des conduits de l'installation.

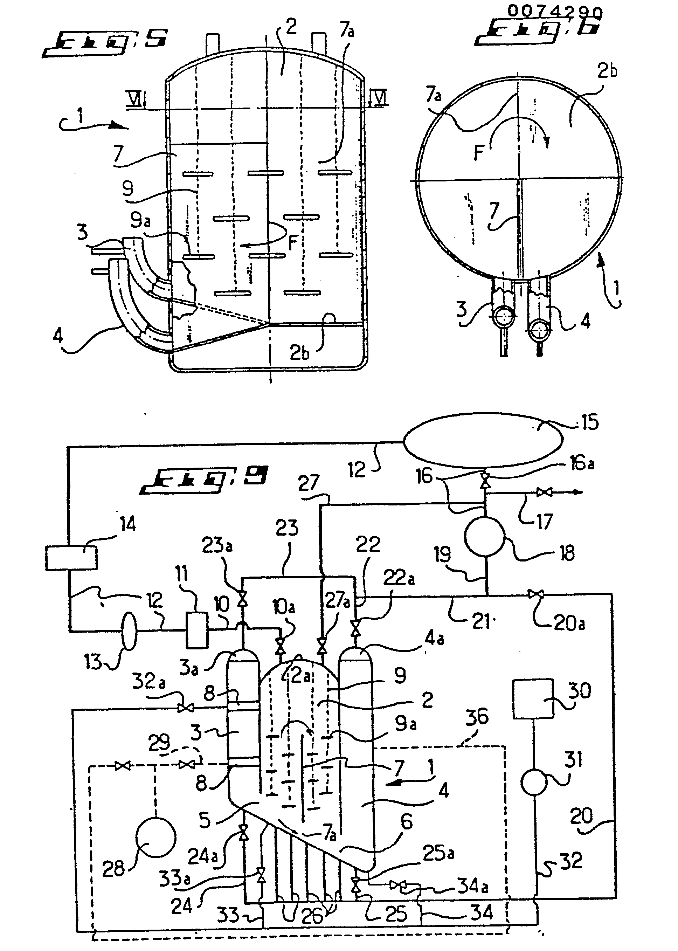

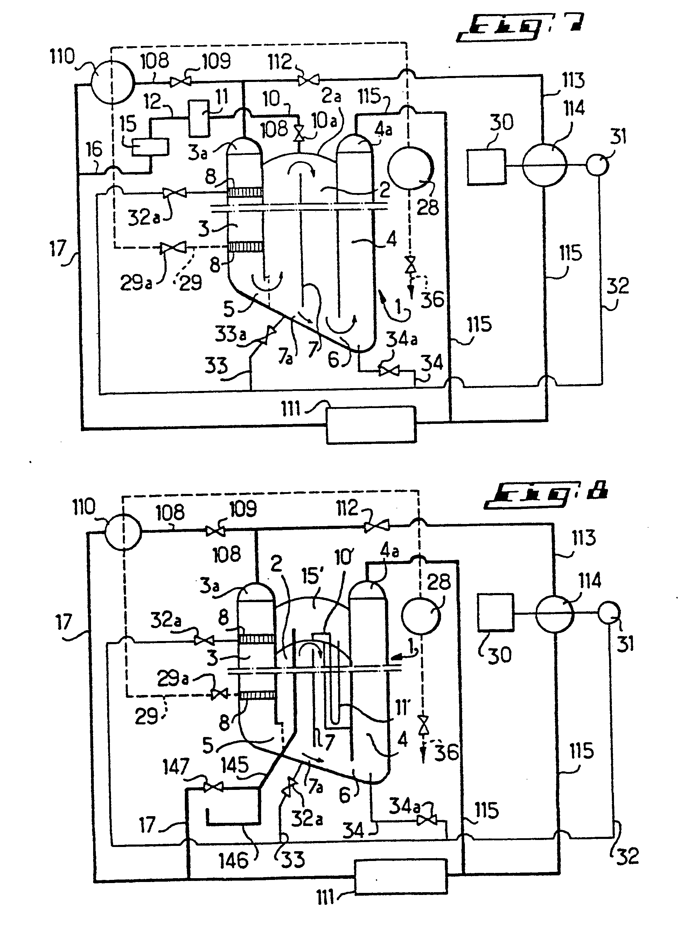

Le second mode de réalisation du réacteur 1 de l'invention illustré aux figures 3 et 4 se différencie du premier mode dans le fait que la valve hydraulique 11'est disposée dans la cuve 2 du réacteur, cette dernière étant surmontée d'un gazomètre 15'. La valve 11'se trouve sur un conduit 10' dont une extrémité part, comme le tuyau 10 de la figure 1, du sommet 2a de la cuve et dont l'autre extrémité débouche dans le gazomètre 15'. Dans ce dernier débouche un tuyau 145 qui aboutit d'une part à un siphon de condensation 146 destiné à récupérer l'eau présente dans les gaz chauds ou biogaz provenant de la fermentation et qui se condense dans le tuyau 145 et d'autre part dans un conduit pour amener le biogaz produit, par exemple, dans un épurateur, une vanne 147 étant prevue sur le conduit 145.

Dans les deux modes de réalisation décrits le fond du réacteur 2b présente une pente unique et le siphon d'entrée 5 est situé à un niveau supérieur à celui du siphon 6.

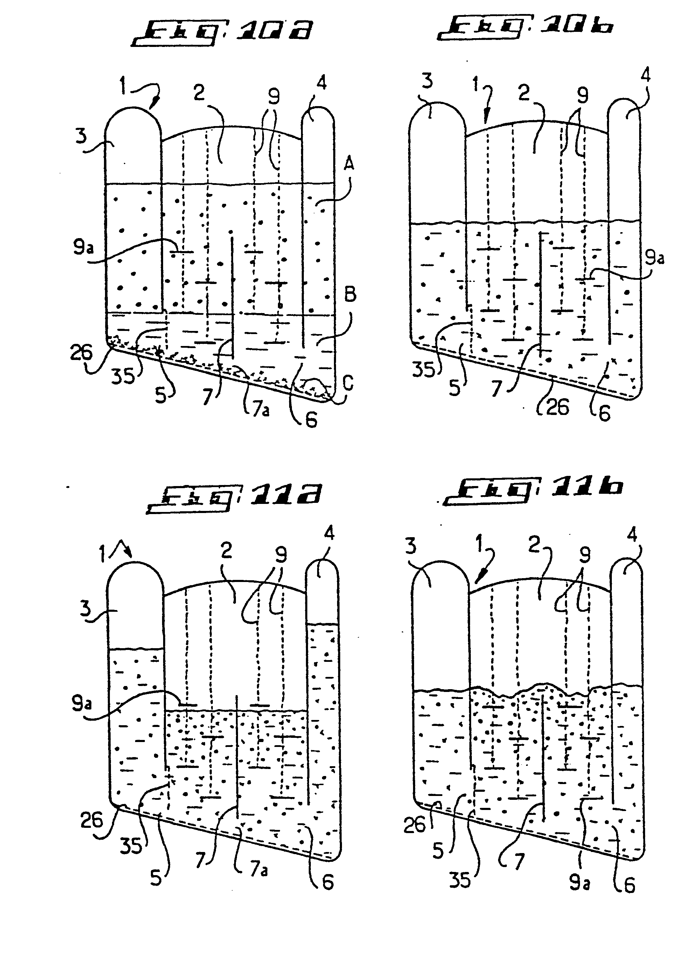

Le mode de réalisation du réacteur illustré aux figures 5 et 6 diffère des deux modes de réalisation précédents en ce que les puits 3 et 4 du réacteur 1 sont disposés à proximité l'un de l'autre sur le pourtour de la cuve 2. La cloison 7 est alors disposée entre l'entrée des deux siphons 5,6 dans la cuve 2 de fermentation, sa largeur est inférieure à la largeur du réacteur pour permettre au substrat de circuler selon la flèche F. De plus, comme dans les deux modes de réalisation, la hauteur de la cloison 7 est inférieure à celle de la cuve 2. Dans ce mode de réalisation, le siphon 5 associé au puits d'alimentation 3 est également situé à un niveau supérieur à celui du siphon 6 associé au puits d'évacuation 4. Le fond 2b de la cuve de fermentation 2 présente une double pente et a avantageusement une forme en ellipse.

Par ailleurs, les chaînes 9 attachées au dôme 2a de la cuve 2 portent, avantageusement, des éléments pesants 9a tels que des disques métalliques, ces disques étant accrochés aux chaînes 9 de préférence à la partie inférieure de celles-ci. Ainsi, les chaînes 9 sont suspendues au dôme 2a de la cuve de manière à pendre librement dans la cuve pour être noyées au moins partiellement dans le substrat à traiter comme cela est clairement illustré aux figures 10a, 10b; 11a, 11b. Il estbien entendu possible de disposer ces chaînes 9 avec les éléments pesants 9a dans le réacteur illustré aux figures 1 à 4.

En outre, selon une autre variante de l'invention, le siphon de sortie 127, le déversoir 128 prévus sur le puits d'évacuation 4 peuvent être remplacés par un conduit d'évacuation équipé avec une vanne (non illustré).

On décrira maintenant en se référant aux figures 7 à 9 l'installation de l'invention et notamment les différents conduits de fluides.

Pour plus de clarté, on a représenté, dans ces figures, les conduits dans lesquels circule le biogaz par un trait fort, les conduits dans lesquels circule le levain ou inoculum en trait fin et les conduits dans lesquels circule l'air par un trait pointillé.

Dans l'installation, illustrée à la figure 9, la cuve de fermentation 2 est reliée à la valve hydraulique 11 par le conduit 10 de sortie des gaz, équipée avantageusement d'une vanne 10a. La valve hydraulique 11 est elle-même reliée au récipient formant gazomètre 15 par un conduit 12 sur lequel sont montés, par exemple, un compteur à gaz 14 et un récipient 13 formant gazomètre-tampon.

Le gazomètre 15 est équipé avec une conduite de sortie 16 munie d'une vanne 16a, qui est connectée d'une part à un conduit 17 pour canaliser le gaz vers, par exemple, un épurateur, une station de stockage, ou un brûleur, et d'autre part à l'entrée d'un compresseur ou surpresseur de gaz 18 pour permettre une circulation du biogaz dans l'installation. La sortie 19 de ce compresseur ou surpresseur 18 de gaz est reliée par un conduit 21 et des dérivations 22 et 23, à travers des vannes 22a, 23a à la partie supérieure des puits d'alimentation et d'évacuation 3, 4. La sortie 19 du surpresseur 18 est également reliée par la conduite 20 équipée d'une vanne 20a à la partie inférieure des puits d'évacuation et d'alimentation 4, 3 par les conduits 24, 25 et à la partie inférieure de la cuve 2 par une pluralité de conduits 26. D'autre part, la partie supérieure de la cuve de fermentation 2 est reliée à l'entrée du compresseur 18 par un conduit 27 équipé d'une vanne 27a.

De préférence, les conduits 26 débouchant dans le fond de la cuve de fermentation 2 et les conduits 24,25 sont équipés avec des clapets anti-retour (non représenté) pour éviter que des matières contenues dans le réacteur 1 chutent dans lesdits conduits. De plus, les conduits 26 peuvent éventuellement être munis de moyens (non représenté) en forme de soupape permettant d'émettre des jets brefs et successifs sous pression.

Le circuit de gaz réprésenté à la figure 9 décrit ci-dessus est donné simplement à titre d'exemple. Il est bien entendu possible de prévoir d'autres conduits ou appareils, vannes ou analogues pour permettre de réaliser tous les circuits possibles pour la circulation du gaz dans le réacteur, et par exemple de prévoir des échanges de chaleur avec des autres fluides de l'installation comme cela est illustré aux figures 7 et 8.

Dans ces figures, le circuit de recirculation du biogaz dans le réacteur n'est pas représenté. Une fraction du biogaz récupérée dans le gazomètre 15 est alimentée directement dans l'épurateur 111 tandis qu'une autre fraction est mise en échange thermique avec l'air éventuellement alimenté dans le puits d'alimentation 3, dans l'échangeur de chaleur 110 puis par les conduits 108,113 et vannes 109,112 subit également un échange thermique dans l'échangeur de chaleur 114 avec le levain recyclé dans le réacteur 1. Cette fraction du biogaz est ensuite alimentée dans l'épurateur 111 par la canalisation 115. Avantageusement, le biogaz présent à la partie supérieure des puits d'alimentation et d'évacuation est alimenté dans l'épurateur 111.

Les circuits de levain ou inoculum et d'air seront décrits simultanément au fonctionnement de l'installation.

Des produits, sous-produits ou déchets organiques d'origine humaine, animale ou végétale qui après avoir éventuellement subi un prétraitement mécanique, physique, chimique ou microbiologique, par exemple un traitement thermique, un broyage ou un hachage par hâcheur projecteur, une dépressurisation rapide, une préfermentation anaérobie, pour favoriser par exemple l'hydrolyse de la matière, la défibrillisation ou la délignification notamment dans le cas de composés cellulosiques et lignocellulosiques sont introduit après ouverture du couvercle 3a dans le puits d'alimentation 3. Le couvercle est ensuite refermé de manière étanche. On introduit alors un gaz pour former une pression de gaz à la partie supérieure du puits d'alimentation 3, par exemple par injection d'air comprimé produit par le compresseur 28, par le conduit 29 à la partie inférieure du puits d'alimentation 3, ou introduction de biogaz par la conduite 23 en ouvrant la vanne 23a. La pression de gaz règnant à la partie supérieure du puits d'alimentation 3 pousse les produits ou substrat à travers le siphon 5 pour l'introduire dans la première partie de la cuve de fermentation 2.

L'injection d'air dans le puits d'alimentation 3 est réalisée si l'on désire oxygéner le substrat alimenté ou provoquer ou poursuivre une fermentation aérobie thermogène du substrat, en fonction de la nature du substrat à traiter.

Le substrat est ainsi introduit dans la première partie de la cuve de fermentation 2 et par un mouvement ascendant se déverse dans la seconde partie de la cuve de fermentation 2 par débordement au-dessus de la cloison 7, puis après un mouvement descendant passe à travers le siphon 6 pour remonter dans le puits d'évacuation 4 où une partie sera évacuée périodiquement par le siphon 127 ou par ouverture du couvercle 4a. Il est également avantageux d'injecter de l'air dans le puits d'évacuation 4 par le conduit 36 pour accélérer le processus de transformation du substrat fermenté en vue de son utilisation en compost.

De manière connue en soi et avantageusement, le substrat recueilli à la sortie du puits d'évacuation 4 est alimenté par un déversoir 130 dans un pressoir 138 qui sépare la partie solide fermentée du liquide ou levain et qui peut éventuellement être soumis à une insufflation d'air. Un tapis transporteur 139 évacue la matière solide tandis que le liquide est récupéré dans un cuvon 30. Ce levain ou inoculum ainsi recueilli peut être utilisé de différentes façons et peut être recyclé sous l'action d'une pompe 31, après avoir été éventuellement réchauffé dans l'échangeur de chaleur 114, dans les différentes parties du réacteur 1. Ainsi, il peut:

- être recyclé dans la partie supérieure du puits d'alimentation 3 par le conduit 32, et notamment si un prétraitement aérobie thermogène est réalisé dans le puits d'alimentation 3, ou
- être injecté dans le bas de la cuve 2 de fermentation par le conduit 33 ou
- être envoyé dans le bas du puits d'évacuation 4 par le conduit 34.

Ces différents conduits sont avantageusement munis respectivement de vannes 32a,33a et 34a.

La nature du substrat traité dans une telle installation peut être très variable.

De plus, l'installation et le procédé de l'invention permettent de conduire une fermentation anaérobie de produits organiques, et notamment une méthanogenèse aussi bien avec un substrat ayant une teneur faible en matières sèches ou un substrat ayant une teneur élevée en matières sèches, de l'ordre de 30% par exemple ou plus, et très hétérogène ou non.

En effet, en se référant à la figure 10a, sans agitation et homogénéisation du substrat dans le réacteur, on obtiendra très rapidement une première couche A chargée de gaz, et une couche inférieure B liquide, des matières lourdes C ayant décanté sur le fond du réacteur. Il se formera alors dans la couche A une croûte ou chapeau qui obstrue le réacteur empêchant toute circulation du substrat et bloquant ainsi le fonctionnement continu du digesteur en provoquant l'arrêt de la fermentation.

Selon l'invention, pour favoriser ie processus de dégradation anaérobie dans le réacteur, on réalise une homogénéisation et une fluidisation du substrat en utilisant le biogazproduit. Cette homogénéisation et fluidisation sont, dans un mode de réalisation de l'invention, réalisées par la conjugaison de trois effets liés à la recirculation du biogaz dans le substrat, au mouvement de flux et reflux du substrat entre la cuve de fermentation et les puits d'alimentation 3 et/ou d'évacuation 4 et enfin au mouvement des chaînes 9 et disques 9a.

A la figure 10b, on a illustré schématiquement l'état du substrat lorsque s'effectue simplement une recirculation ou bullage du biogaz dans ledit substrat, Cette injection de biogaz dans le substrat est réalisée au moyen du compresseur 18 soit à travers les conduits 22, 23, soit les conduits 24, 25 soit les conduits 26 ou par un ou plusieurs de ces conduits par l'ouverture ou la fermeture des vannes 20a, 22a, 23a, 24a, 25a.

Toutefois, cette recirculation de biogaz, si elle est réalisée isolément est surtout efficace pour le traitement d'un substrat à faible teneur en matières sèches permettant une dispersion des produits lourds C et une meilleure répartition des calories apportées au réacteur 1.

Aux figures 11a, 11b, on a illustré l'état du substrat dans le cas où l'on applique les trois effets combinés de la recirculation du gaz, du mouvement du flux et de reflux du substrat et des chaînes 9.

Le mouvement de flux et reflux de la masse du substrat entre la cuve 2 et les puits d'alimentation et d'évacuation 3, 4, est obtenu au moyen de la valve hydraulique 11 qui permet d'élever la pression du biogaz contenu au-dessus du substrat dans la cuve de fermentation 2. Pendant cette période, le substrat est refoulé dans le puits d'évacuation 4 et/ou dans le puits d'alimentation 3 (figure 11a). Lorsque le seuil de pression est atteint, la valve hydraulique 11 permet le dégagement du biogaz vers le gazomètre-tampon 13, la pression règnant dans la cuve de fermentation 2 chute brutalement et provoque un reflux de substrat dans ladite cuve de fermentation pour revenir à son niveau initial (figure 11b). Par ailleurs, les mouvements de flux et reflux du substrat provoquent le mouvement des chaînes 9 maintenues rigides par les éléments pesants 9a, ce qui casse la surface supérieure ou chapeau du substrat qui se forme au cours de la méthanisation. L'humidification de ce chapeau brisé s'effectue aussitôt ce qui évite la formation par déshydratation d'une croûte qui peut colmater à la longue le réacteur. De plus, les chaînes 9 et disques 9a forment des chemins préférentiels pour le dégagement du biogaz dans le substrat.

Ainsi, sous l'effet combiné de la recirculation du biogaz, de la valve hydraulique et des chaînes et disques, on obtient une agitation importante du substrat qui permet sa fluidisation quand celui-ci à une teneur élevée en matières sèches. Par ailleurs, la recirculation du biogaz permet d'éviter l'accumulation de matières lourdes décantées au fond du réacteur. Ainsi, on peut extraire par le puits d'évacuation 4 un substrat en forme de pâte épaisse fortement chargée en matières sèches.

Le procédé et l'installation de l'invention permet donc de traiter dans des conditions très favorables pour une bonne dégradation anaérobie aussi bien un substrat à faible teneur en matières sèches qu'un substrat à forte teneur en matières sèches. Ainsi, il est possible d'une part de diminuer fortement les volumes des cuves de fermentation et d'autre part d'assurer de meilleures conditions de déroulement des différentes phases formant les processus de la digestion anaérobie. En effet, un substrat fortement chargé en matières sèches est un très bon support physiques des populations microbiologiques nécessaires à la digestion anaérobie, et son pouvoir tampon est amélioré au cours de la fermentation. De plus, la concentration élevée de matières organiques à l'intérieur du réacteur permet d'améliorer la production de gaz, et par une meilleure fixation de la biomasse de rendre les procédés de digestion fiables et efficaces.

Bien entendu, selon la nature du substrat il est possible d'utiliser simplement l'effet de la valve hydraulique combinée avec les chaînes 9, ou simplement la recirculation de biogaz.

Par ailleurs, la poussée pneumatique des produits dans la cuve 2 de fermentation à travers un siphon, et leur évacuation également à travers un siphon permet d'alimenter périodiquement le substrat à traiter dans le puits d'alimentation 3 et d'extraire périodiquement le substrat fermenté par le puits d'évacuation 4 tout en maintenant constamment une masse de substrat dans la cuve de fermentation 2 pour ne pas bloquer le fonctionnement continu du digesteur en provoquant l'arrêt de la fermentation.

En outre, le réacteur 1 est avantageusement muni d'un système de chauffage qui maintiendra la cuve à la température voulue pour favoriser le processus de dégradation anaérobie, ce système de chauffage peut être, par exemple intégré dans le double fond de la cuve illustré à la figure 5. De plus, le réacteur 1 et les différentes canalisations sont parfaitement isolés thermiquement pour limiter les pertes d'énergie. A cet effet, on prévoit différents échangeurs de chaleur entre les fluides de l'installation comme illustré aux figures 7 et 8 ou, par exemple, de réchauffer le levain ou inoculum par passage dans un digesteur situé en amont du réacteur 1 dans lequel est réalisé un pretraitement du substrat par dégradation aérobie thermogène, pour ainsi optimiser le rendement énergétique de l'installation.

De plus, l'installation comprend, de manière connue, et avantageusement, des appareils pour les mesures physico-chimiques nécessaires, telles que le débit de gaz, la pression, la température, le pH, et le rH et la teneur en oxygène.

## Revendications

1. Procedé pour réaliser une dégradation en milieu anaérobie, par exemple une méthanogénèse, de produits, sous produits ou déchets organiques d'origine humaine, animale et/ou végétale avantageusement à teneur élevée en produits solides, consistant à alimenter lesdits produits à dégrader dans une enceinte fermée, après avoir éventuellement ensemencé lesdits produits avec un substrat convenable, à imposer auxdits produits un sens de circulation dans ladite enceinte, à recueillir le gaz produit appelé biogaz dégagé au-dessus de ladite masse de produits, et à évacuer les produits dégradés, caractérisé en ce qu'on réalise une réintroduction du biogaz dans ladite enceinte pour aboutir à une agitation et/ou homogénéisation et/ou circulation forcée desdits produits.

2. Procédé selon la revendication 1, caractérisé en ce que l'alimentation des produits se fait pneumatiquement en exerçant une poussée pneumatique sur lesdits produits pour les forcer à pénètrer dans l'enceinte, en réalisant ainsi une circulation dirigée et forcée desdits produits.

3. Procédé selon la revendication 1 ou 2, caractérisé en ce que l'alimentation se fait à travers un puits d'alimentation, la poussée pneumatique précitée est obtenue par injection de gaz sous pression, par exemple de l'air comprimé ou du biogaz dans ledit puits d'alimentation.

4. Procédé selon la revendication 2, caractérisé en ce que la poussée pneumatique est réalisée par injection de biogaz sous pression.

5. Procédé selon l'une quelconque des revendications précédentes, caractérisé en ce qu'on effectue un prétraitement des produits avant leur alimentation dans l'enceinte précitée, ce prétraitement étant soit un traitement mécanique et/ou thermique, et/ou microbiologique en milieu aérobie.

6. Procédé selon l'une quelconque des revendications précédentes, caractérisé en ce qu'on réalise des échanges de chaleur entre l'air, le biogaz et/ou le levain.

7. Procédé selon l'une quelconque des revendications précédentes, caractérisé en ce qu'on réalise une réintroduction du biogaz dans la masse de produits présents dans ladite enceinte, de préférence par émission de jets successifs sous pression.

8. Installation pour réaliser une dégradation en milieu anaérobie par exemple méthanogénèse, de produits, sous-produits ou déchets organiques d'origine humaine, animale ou végétale, du type comprenant un réacteur ou digesteur ayant une cuve de fermentation anaérobie divisée verticalement en deux parties, une première partie étant reliée à un puits d'alimentation, la seconde partie étant reliée à un puits d'évacuation des produits fermentés, une sortie de gaz ou biogaz reliée à un récipient formant gazomètre à travers une valve appropriée, et des moyens reliés audit puits d'évacuation pour recueillir les produits fermentes, caractérisée en ce qu'elle comprend des moyens de réintroduction de biogaz sous pression dans ladite enceinte de manière à aboutir à une agitation et/ou homogénéisation et/ou circulation forcée desdits produits.

9. Installation selon la revendication 8, caractérisée en ce que les moyens précités de réintroduction de biogaz comprennent un compresseur (18) dont l'entrée (16) est au moins reliée au gazomètre (15) précité et dont la sortie (19) est reliée à une pluralité de conduits (26) débouchant dans le fond de la cuve de fermentation (2), ou à un conduit (22) débouchant au sommet du puits d'alimentation (3) ou à un conduit (24) débouchant dans le fond du puits d'alimentation (3), de manière à réaliser une poussée pneumatique des produits.

10. Installation selon la revendication 9, caractérisée en ce que la sortie (19) précitée du compresseur (18) est reliée à un conduit (23) débouchant au sommet du puits d'évacuation (4) ou à un conduit (25) débouchant dans le fond du puits d'évacuation (4).

11. Installation selon l'une des revendications 8 à 10, caractérisée en ce que la valve de gazomètre précitée peut être ouverte par intermittence, ladite valve étant de préférence une valve hydraulique (11) de manière à provoquer des variations brutales de la pression de gaz contenue dans l'enceinte fermée.

12. Installation selon l'une des revendications 8 à 11, caractérisée en ce qu'elle comprend un compresseur à gaz (18) ou à air (28) dont la sortie est reliée au puits d'alimentation (3) précité et/ou au puits d'évacuation (4) precité.

13. Installation selon l'une des revendications 8 à 12, caractérisée en ce qu'elle comprend des moyens (30, 31) pour recycler le liquide séparé des produits solides fermentés recueillis à la sortie du puits d'évacuation (4) précité, dans la cuve de fermentation (2) précitée et/ou dans le puits d'alimentation (3) ou le puits d'évacuation (4) précité.

14. Installation selon l'une des revendications 7 à 13, caractérisée en ce que la cloison précitée (7) séparant la cuve (2) de fermentation en des première et seconde parties, présente un passage (7a) formant communication entre lesdites première et seconde parties, au moins au niveau du fond de la cuve.

15. Installation selon l'une des revendications 7 à 14, caractérisée en ce que le puits d'alimentation precité possède deux séries de fentes (8) situées à une distance l'une de l'autre et à une certaine distance, respectivement, du couvercle (2a) et de l'extrémité inférieure du puits, l'air en provenance du compresseur (28) précité s'écoulant vers le haut du puits d'alimentation (3) à travers la série de fentes inférieures puis à travers la série de fentes supérieure.

16. Installation selon l'une des revendications 7 à 15, caractérisée en ce qu'un premier siphon (5) relie le puits d'alimentation (3) à la cuve de fermen-tation(2) et est équipé avec des moyens pour freiner le retour du substrat dans ledit siphon, tels que par exemple des chaînes à pliage unidirectionnel (35).

17. Installation selon l'une des revendications 7 à 15, caractérisée en ce que les puits d'alimentation et d'évacuation (3, 4) précités sont situés à proximité l'un de l'autre sur le pourtour de la cuve de fermentation (2) précitée, la cloison précitée (7) étant disposée verticalement entre les entrées d'un premier (5) et d'un second siphon (6) et a une largeur inférieure à la largeur de la cuve de fermentation (2) et une hauteur inférieure à celle de la cuve (2).

18. Installation selon l'une des revendications 7 à 17, caractérisée en ce que le fond de la cuve de fermentation présente une double pente et a avantageusement une forme d'éllipse.

19. Installation selon l'une des revendications 7 à 18, caractérisée en ce que les puits d'alimentation et d'évacuation (3, 4) précités sont disposés de manière sensiblement diamétralement opposés sur le pourtour de la cuve de fermentation (2) précitée, la cloison (7) étant disposée verticalement et selon sensiblement un diamètre de la cuve (2) et a une hauteur inférieure à celle de la cuve (2).

20. Installation selon la revendication 19, caractérisée en ce que le fond de la cuve (2) a une pente unique.

21. Installation selon la revendication 19 ou 20, caractérisée en ce que la cloison (7) précitée présente un passage à sa partie inférieure.

22. Installation selon l'une des revendications 7 à 21, caractérisée en ce que la cuve de fermentation anaérobie (2) contient la valve (11') notamment hydraulique destinée à provoquer les oscillations de la masse en fermentation et supportant un gazomètre (15') d'où le gaz peut s'échapper par un tuyau (145) qui traverse la cuve (2) et aboutit d'une part à un siphon de condensation (146) destiné à récupérer l'eau présente dans le biogaz provenant de la fermentation et d'autre part à un épurateur (111), par exemple.

23. Installation selon l'une des revendications 7 à 22, caractérisée en ce qu'elle comprend des échangeurs de chaleur (110, 114) pour réaliser un échange thermique entre le biogaz produit et respectivement l'air produit par le compresseur (28) précité ou le levain précité récupéré.

24. Installation selon l'une des revendications 7 à 23, caractérisée en ce que les conduits (26) précités débouchant dans le fond de la cuve de fermentation (2) precitée comprennent chacun un clapet anti-retour et éventuellement une soupape pour insuffler le gaz par jets brefs et successifs.

**Claims**

1. Method for carrying out a degradation in an anaerobic medium, for instance a methanogenesis, of organic products, byproducts or waste of human, animal and/or vegetable origin advantageously with a high content in solid substances, consisting in feeding the said products to be degraded into a closed enclosure after having possibly sowed the said products with a suitable substrate, imposing on the said products a direction of circulation in the said enclosure, collecting the gas produced called biogas evolved above the said mass of products and discharging the degraded products, characterized in that one performs a re-introduction of the biogas into the said enclosure to result in a forced stirring and/or homogenization and /or circulation of the said products.

2. Method according to claim 1, characterized in that the supply of the products is made pneumatically by exerting a pneumatic thrust upon the said products to force them to penetrate into the enclosure thereby carrying out a guided and forced circulation of the said products.

3. Method according to claim 1 or 2, characterized in that the supply is effected through a a feed snaft, the aforesaid pneumatic thrust is obtained through injection of gas under pressure, for instance of compressed air or of the biogas into the said feed shaft.

4. Method according to claim 2, characterized in that the pneumatic thrust is performed through injection of biogas under pressure.

5. Method according to any one of the foregoing claims, characterized in that one effects a pretreatment of the products before their feeding into the aforesaid enclosure, this pretreatment being either a mechanical and/ or therm.l and /or microbiological treatment in an aerobic medium.

6. Method according to any one of the foregoing claims, characterized in that one carries out heat exchanges between the air, the biogas and/or the leaven.

7. Method according to any one of the foregoing claims, characterized in that one performs a re-introduction of the biogas into the mass of products present within the said enclosure, preferably through emission of successive jets under pressure.

8. Plant for carrying out a degradation in an anaerobic medium, for instance a methanogenesis, of organic products, byproducts or waste of human, animal or vegetable origin, of the type comprising a reactor or digester having an anaerobic fermenting vat vertically divided into two parts, a first part being connected to a feed shaft, the second part being connected to a shaft for discharging tha fermented products, a gas or biogas outlet connected to a container forming a gasometer through a suitable valve, and means connected to the said discharge shaft for collecting the fermented products, characterized in that it comprises means for re-introducing biogas under pressure into the said enclosure so as to result in a forced stirring and/or homogenization and/or circulation of the said products.

9. Plant according to claim 8, characterized in that the aforesaid biogas re-introducing means comprise a compressor (18) the inlet (16) of which is at least connected to the aforesaid gasometer (15) and the outlet (19) of which is connected to a plurality of ducts (26) opening into the bottom of the fermenting vat (2) or to a duct (22) opening at the top of the feed shaft (3) or to a duct (24) opening into the bottom of the feed shaft (3), so as to carry out a pneumatic drive of the products.

10. Plant according to claim 9, characterized in that the aforesaid outlet (19) of the compressor (18) is connected to a duct (23) opening at the top of the discharge shaft (4) or to a duct (25) opening into the bottom of the discharge shaft (4).

11. Plant according to one of claims 8 to 10, characterized in that the aforesaid gasometer valve may be opened intermittently, the said valve preferably being a hydraulic valve (11), so as to induce sudden variations in the gas pressure contained within the closed enclosure.

12. Plant according to one of claims 8 to 11, characterized in that it comprises a gas or air compressor (18, 28) the outlet of which is connected to the aforesaid feed shaft (3) and /or to the aforesaid discharge shaft (4).

13. Plant according to one of claims 8 to 12, characterized in that it comprises means (30, 31) for recycling the liquid separated from the fermented solid products collected at the outlet of the aforesaid discharge shaft (4), into the aforesaid fermenting vat (2) and/or into the aforesaid feed shaft (3) or discharge shaft (4).

14. Plant according to one of claims 7 to 13, characterized in that the aforesaid partition (7) separating the fermenting vat (2) into first and second parts exhibits a passage-way (7a) forming a communication between the said first and second parts, at least at the level of the bottom of the vat.

15. Plant according to one of claims 7 to 14, characterized in that the aforesaid feed shaft (3) has two series of slots (8) located at a distance from each other and at a certain distance, respectively, from the cover (2a) and from the lower end of the shaft, the air, coming from the aforesaid compressor (28), flowing upwards of the feed shaft (3) through the series of lower slots and then through the series of upper slots.

16. Plant according to one of claims 7 to 15, characterized in that a first siphon (5) connects the feed shaft (3) to the fermenting vat (2) and is fitted with means for braking the return of the substrate into the said siphon, such for instance as unidirectionally foldable chains (35).

17. Plant according to one of claims 7 to 15, characterized in that the aforesaid feed and discharge shafts (3, 4) are located in proximity of each other on the periphery of the aforesaid fermenting vat (2), the aforesaid partition (7) being arranged vertically between the inlets of a first (5) and of a second siphon (6) and has a width smaller than the width of the fermenting vat (2) and a height smaller than that of the vat (2).

18. Plant according to one of claims 7 to 17, characterized in that the bottom of the fermenting vat exhibits a double slope and advantageously has the shape of an ellipse.

19. Plant according to one of claims 7 to 18, characterized in that the aforesaid feed and discharge shafts (3, 4) are arranged in substantially diametrally opposite relationship on the periphery of the aforesaid fermenting vat (2), the partition (7) being positioned vertically and substantially along a diameter of the vat (2) and has a height smaller than that of the vat (2).

20. Plant according to claim 19, characterized in that the bottom of the vat (2) has a single slope.

21. Plant according to claim 19 or 20, characterized in that the aforesaid partition (7) exhibits a passageway at its lower portion.

22. Plant according to one of claims 7 to 21, characterized in that the anaerobic fermenting vat (2) contains the in particular hydraulic valve (11') adapted to induce the oscillations of the mass being in the process of fermenting and supporting a gasometer (15') from where the gas may escape through a pipe (145) which extends through the vat (2) and leads on the one hand to a condensing siphon (146) adapted to recover the water present in the biogas evolving from the fermentation and on the other hand to a cleaner (111) for instance.

23. Plant according to one of claims 7 to 22, characterized in that it comprises heat exchangers (110, 114) to perform a thermal exchange between the biogas produced and the air produced by the aforesaid compressor (28) or the aforesaid recovered leaven, respectively.

24. Plant according to one of claims 7 to 23, characterized in that the aforesaid ducts (26) opening into the bottom of the aforesaid fermenting vat (2) comprise each one a check valve and possibly a valve for blowing the gas in through short and successive jets.

**Patentansprüche**

1. Verfahren zur Durchführung eines Abbaues im anaeroben Medium z B. einer Methanentstehung, von organischen Erzeugnissen, Nebenerzeugnissen oder Abfällen menschlicher, tierischer und/oder pflanzlicher Herkunft, in vorteilhafter Weise mit einem hohen Feststoffgehalt, welches darin besteht, die abzubauenden besagten Erzeugnisse in einen geschlossenen Raum einzuführen, nach etwaiger Einimpfung der besagten Erzeugnisse mit einem geeigneten Substrat, den besagten Erzeugnissen eine Strömungrichtung in dem besagten geschlossenen Raum aufzuerlegen, das oberhalb der gesagten Masse von Erzeugnissen entwickelte, Biogas genannte, erzeugte Gas aufzufangen und die abgebauten Erzeugnisse abzuführen, dadurch gekennzeichnet, dass man eine Wiedereinführung des Biogases in den besagten geschlossenen Raum bewirkt,um eine zwangsläufige Rührbewegung und/oder Homogenisierung und/oder Strömung der besagten Erzeugnisse zu ergeben.

2. Verfahren nach Anspruch 1, dadurch gekennzeichnet, dass die Zufuhr der Erzeugnisse pneumatisch geschieht, indem ein pneumatischer Schub auf die besagten Erzeugnisse ausgeübt wird, um diese zu zwingen, in den geschlossenen Raum einzudringen, wobei somit eine gesteuerte und zwangsläufige Strömung der besagten Erzeugnissen bewirkt wird.

3. Verfahren nach Anspruch 1 oder 2, dadurch gekennzeichnet, dass die Zufuhr durch einen Zufuhrschacht erfolgt und der vorgenannte pneumatische Schub durch Einspritzung von Gas unter Druck, z.B. von Druckluft oder des Biogases in den besagten Zufuhrschacht erzielt wird.

4. Verfahren nach Anspruch 2, dadurch gekennzeichnet, dass der pneumatische Schub durch Einspritzung von Biogas unter Druck bewirktwird.

5. Verfahren nach irgendeinem der vorangehenden Ansprüche, dadurch gekennzeichnet, dass man eine Vorbehandlung der Erzeugnisse vor deren Einführung in den besagten geschlossenen Raum durchführt, wobei diese Vorbehandlung entweder eine mechanische und/oder thermische und/oder mikrobiologische Vorbehandlung im aeroben Medium ist.

6. Verfahren nach irgendeinem der vorangehenden Ansprüche, dadurch gekennzeichnet, dass man Wärmetausche zwischen der Luft, dem Biogas und/oder dem Treibmittel durchführt.

7. Verfahren nach irgendeinem der vorangehenden Ansprüche, dadurch gekennzeichnet, dass man eine Wiedereinführung des Biogases in die Masse der in dem besagten geschlossenen Raum vorhandenen Erzeugnisse vorzugsweise durch Aussendung von aufeinanderfolgenden Strahlen unter Druck durchführt.

8. Anlage zur Durchführung eines Abbaues im anaeroben Medium, z.B. einer Methanentstehung, von organischen Erzeugnissen, Nebenerzeugnissen oder Abfällen menschlicher tierischer oder pflanzlicher Herkunft, derjenigen Bauart mit einem Reaktor bzw. Digerierapparat, der eine lotrecht in zwei Teilen unterteilte anaerobe Gärungsküpe hat, wobei ein erster Teil mit einem Zufuhrschacht verbunden ist, wobei der zweite Teil mit einem Abfuhrschacht für die vergorenen Erzeugnisse verbunden ist, einem über ein geeignetes Ventil mit einem einen Gasometer bildenden Gefäss verbundenen Gas- bzw. Biogasauslass, und mit dem besagten Abfuhrschacht verbundenen Mitteln, um die vergorenen Erzeugnisse aufzufangen, dadurch gekennzeichnet, dass sie Mittel zur Wiedereinführung von Biogas unter Druck in den besagten geschlossenen Raum umfasst, um eine zwangsläufige Rührbewegung und/oder Homogenisierung und/oder Strömung der besagten Erzeugnisse zu erzielen.

9. Anlage nach Anspruch 8, dadurch gekennzeichnet, dass die vorgenannten Mittel zur Wiedereinführung von Biogas einen Verdichter (18) aufweisen, dessen Einlass (16) ist und dessen Auslass (19) mit einer Mehrzahl von in den Boden von der Gärungsküpe (2) mündenden Leitungen (26) oder mit einer am Kopf des Zufuhrschachts (3) mündenden Leitung (22) oder mit einer in den Boden des Zufuhrschachts (3) mündenden Leitung (24) verbunden ist, um somit einen pneumatischen Vorschub der Erzeugnisse zu bewirken.

10. Anlage nach Anspruch 9, dadurch gekennzeichnet, dass der vorgenannte Auslass (19) des Verdichters (18) mit einer am Kopf des Abfuhrschachtes (4) mündenden Leitung (23) oder mit einer in den Boden des Abfuhrschachtes (4) mündenden Leitung (25) verbunden ist.

11. Anlage nach einem der Ansprüche 8 bis 10, dadurch gekennzeichnet, dass das vorgenannte Gasometerventil besagte Ventil vorzugsweise ein hydraulisches Ventil (11) ist, um plötzliche Änderungen des in dem geschlossenen Raum enthaltenen Gasdrucks hervorzurufen.

12. Anlage nach einem der Ansprüche 8 bis 11, dadurch gekennzeichnet, dass sie einen Gas- oder Luftverdichter (18, 28) umfasst, dessen Auslass an den vorgenannten Zufuhrschacht (3) und / oder an den vorgenannten Abfuhrschacht (4) angeschlossen ist.

13. Anlage nach einem der Ansprüche 8 bis 12, dadurch gekennzeichnet, dass sie Mittel (30, 31) zur Rückführung der von den am Auslass des vorgenannten Abfuhrschachtes (4) gewonnenen, vergorenen Feststofferzeugnissen abgeschiedenen Flüssigkeit in die vorgenannte Gärungsküpe (2) und / oder in den vorgenannten Zufuhrschacht (3) oder Abfuhrschacht (4) aufweist.

14. Anlage nach einem der Ansprüche 7 bis 13, dadurch gekennzeichnet, dass die, die Gärungsküpe (2) in einen ersten und einen zweiten Teil aufteilende vorgenannte besagten ersten und zweiten Teil bildenden Durchgang (7a) wenigstens in der Höhe des Bodens der Küpe aufweist.

15. Anlage nach einem der Ansprüche 7 bis 14, dadurch gekennzeichnet, dass der vorgenannte Zufuhrschacht (3) zwei Reihen im Abstand voneinander und in einem bestimmten Abstand jeweils des Deckels (2a) und des unteren Endes des Schachtes liegenden Schlitzen (8) besitzt, wobei die von dem vorgenannten Verdichter (28) herkommende Luft nach oben des Zufuhrschachtes (3) durch die Reihe der unteren Schlitze und dann durch die Reihe der oberen Schlitze strömt.

16. Anlage nach einem der Ansprüche 7 bis 15, dadurch gekennzeichnet, dass ein erster Siphon (5) den Zufuhrschacht (3) mit der Gärungsküpe (2) verbindet und mit Mitteln zum Abbremsen der Rückfuhr des Substrates in den besagten Siphon, wie z.B. in einer Richtung faltbaren Ketten (35) ausgerüstet ist.

17. Anlage nach einem der Ansprüche 7 bis 15, dadurch gekennzeichnet, dass die vorgenannten Zufuhr- und Abfuhrschachte (3,4) in der Nähe voneinander am Umfang der vorgenannten Gärungsküpe (2) liegen, wobei die vorgenannte Trennwand (7) lotrecht zwischen den Einlässen eines ersten (5) und eines zweiten Siphons (6) angeordnet ist und eine kleinere Breite als die Breite der Gärungsküpe (2) und eine kleinere Höhe als diejenige der Küpe (2) hat.

18. Anlage nach einem der Ansprüche 7 bis 17, dadurch gekennzeichnet, dass der Boden der Gärungsküpe eine Doppelneigung aufweist und in vorteilhafter Weise die Gestalt einer Ellipse hat.

19. Anlage nach einem der Ansprüche 7 bis 18, dadurch gekennzeichnet, dass die vorgenannten Zufuhr- und Abfuhrschachte (3,4) in etwa diametral gegenüberliegender Weise am Umfang der vorgenannten Gärungsküpe (2) angeordnet sind, wobei die Trennwand (7) lotrecht und im wesentlichen entlang eines Durchmessers der Küpe (2) angeordnet ist und eine kleinere Höhe als diejenige der Küpe (2) hat.

20. Anlage nach Anspruch 19, dadurch gekennzeichnet, dass der Boden der Küpe (2) eine einzige Neigung hat.

21. Anlage nach Anspruch 19 oder 20, dadurch gekennzeichnet, dass die vorgenannte Trennwand (7) einen Durchlass an ihrem unteren Teil aufweist.

22. Anlage nach einem der Ansprüche 7 bis 21, dadurch gekennzeichnet, dass die anaerobe Gärungsküpe (2) ein insbesondere hydraulisches Ventil (11') enthält, welches bestimmt ist, die Schwingungen der gärenden Masse zu verursachen und welche einen Gasometer (15') trägt, aus welchem das Gas über eine Rohrleitung (145) entweichen kann, welche die Küpe (2) durchsetzt und einerseits zu einem Kondensierungssiphon (146), der zur Wiedergewinnung des in dem von dem Gärungsvorgang herrührenden Biogas vorhandenen Wassers bestimmt ist und andererseits z.B. zu einem Reiniger (111) führt.

23. Anlage nach einem der Ansprüche 7 bis 22, dadurch gekennzeichnet, dass sie Wärmetauscher (110, 114) aufweist, zur Durchführung eines Wärmetausches zwischen dem erzeugten Biogas und jeweils der durch den vorgenannten Verdichter (28) erzeugten Luft und dem wiedergewonnenen vorgenannten Treibmittel.

24. Anlage nach einem der Ansprüche 7 bis 23, dadurch gekennzeichnet, dass die in den Boden der vorgenannten Gärungsküpe (2) mündenden vorgenannten Leitungen (26) jeweils ein Rückschlagventil und ggf. ein Ventil zum Einblasen des Gases durch kurze aufeinanderfolgende Strahle umfassen.

Fig. 1

Fig. 2

Fig. 12

Fig 4

Fig 3

FIG. 5

FIG. 6

FIG. 9

Fig. 7

Fig. 8

Fig. 10a

Fig. 10b

Fig. 11a

Fig. 11b